# EUROPEAN PATENT SPECIFICATION

(11) **EP 2 171 471 B1**
(45) Date of publication and mention of the grant of the patent: **18.11.2015**
(21) Application number: 08768184.7
(22) Date of filing: 06.06.2008
(51) Int. Cl.: G01N 33/66

(54) **SENSORS FOR THE DETECTION OF DIOLS AND CARBOHYDRATES USING BORONIC ACID CHELATORS FOR GLUCOSE**
SENSOREN ZUM NACHWEIS VON DIOLEN UND KOHLENHYDRATEN MIT VERWENDUNG VON BORONSÄURECHELATOREN FÜR GLUCOSE
DÉTECTEUR POUR LA DÉTECTION DE DIOLS ET D'HYDRATES DE CARBONES UTILISANT DES CHÉLATEURS ACIDE BORONIQUE POUR LE GLUCOSE

(30) Priority: 08.06.2007 US 933724 P; 29.05.2008 US 130195
(43) Date of publication of application: 07.04.2010
(73) Proprietor: The Charles Stark Draper Laboratory, Inc., Cambridge, MA 02139 (US)
(72) Inventor: CLARK, Heather, A., Lexington, MA 02421-5829 (US)
(74) Representative: Vossius & Partner Patentanwälte Rechtsanwälte mbB
(86) International application number: PCT/US2008/007108
(87) International publication number: WO 2008/153930

(56) References cited:
- WO-A-2007/054689
- WO-A-2007/067743
- CLARK H A ET AL: "OPTOCHEMICAL NANOSENSORS AND SUBCELLAR APPLICATIONS IN LIVING CELLS" MIKROCHIMICA ACTA, SPRINGER VERLAG, VIENNA, AT, vol. 131, no. 1/02, 1 January 1999 (1999-01-01), pages 121-128, XP000992957 ISSN: 0026-3672

## Description

Clark et al. (1999) Microchimica Acta 131(1-2):121-128 describes optochemical nanosensors, in more detail optochemical PEBBLE (Probe Encapsulated By Biologically Localized Embedding) sensors which can be used for the monitoring of intracellular analytes.

### BACKGROUND OF THE INTENTION

Diabetes has become a national health-care crisis. According to the 2005 National Diabetes Fact Sheet, an estimated 20.8 million people in the United States suffer from diabetes. The costs associated with diabetic care are also astronomical, with an estimated $132 billion dollars spent in 2002. As a result of a seminal study highlighting the benefits of tight glycemic control, the American Diabetes Association recommends that patients with diabetes should try to control their glucose levels to be as close to normal as possible. With tight glycemic control, the complications associated with diabetes, such as heart disease, blindness and amputation are significantly reduced. Self monitoring of glucose is essential for regulation, particularly for those with Type 1 diabetes. It is often performed through a finger-stick method three times or more per day. The need to draw blood, even in small quantities, multiple times a day is not desirable.

A continuous monitoring system would be highly advantageous for patients and healthcare providers alike. It has become the goal of glucose sensor research, and continuous monitoring systems of many varieties are pursued by countless researchers in the field. The benefits of continuous monitoring over the finger-stick method are numerous. First, the finger-stick method is both painful and inconvenient for the patient, which can lead to noncompliance. Second, a single-point measurement gives static information on the concentration of blood glucose, with no knowledge of the trend, or in other words, whether the level is going up or down. Third, monitoring at night, a time when levels could dip dangerously low, is either not performed or especially inconvenient. Continuous monitoring systems have been pursued in many different forms, and some are commercially available, such as the Guardian RT from Medtronic MiniMed (Northridge, California), and the GlucoWatch Biographer from Animas (West Chester, PA). Both of these systems work by sampling glucose from the interstitial space, the extracellular space in the dermis, rather than the blood. Currently, they are approved as monitors to track trends in glucose but highs and lows are verified by a finger-stick test. Some reports have shed doubt on the accuracy of nighttime monitoring in patients whose glucose is tightly controlled.

Commercially available systems for continuous or finger-stick measurements rely on electrochemical biosensors. Glucose oxidase is the most well-known of the biological recognition units, and the enzyme provides a highly selective sensor platform. Enzyme-based sensors are difficult to implement as implantable glucose sensors, since the enzyme limits itself in a confined environment. Oxygen, required for function, regionally depletes, and hydrogen peroxide, a by-product of the reaction, can lead to enzyme degradation. Most often the read-out is electrode-based, which is an added challenge for miniaturization and biological implantation. Nano- and microscale optical sensors have also been demonstrated, but typically lack the selectivity and robustness to replace traditional techniques.

There is still a need for a continuous, non-invasive method for glucose monitoring, especially one that is easy to use, highly accurate and pain-free.

The present invention is defined by the claims. In more detail, the present invention relates to a sensor particle for detecting the presence of a chelatable analyte, comprising: a quantum dot or fluorescent dye; a polymer matrix comprising a polymer including moieties that bind the chelatable analyte, wherein the chelatable analyte is glucose or fructose; and a chromophore associated with the polymer matrix that binds to the moieties in the absence of the chelatable analyte, wherein the moities that blind glucose or fructose comprise one of boronic acid and boronic ester; and the sensor particle emits photons with an inner filter effect, wherein: photons emitted by the quantum dot or fluorescent dye in an excited state are absorbed by the chromophore in an unbound state but not by the chromophore in a bound state; or photons emitted by the quantum dot or fluorescent dye in an excited state are absorbed by the chromophore in a bound state but not by the chromophore in an unbound state.

### SUMMARY OF THE INVENTION

Also described herein is a sensor particle for detecting the presence of a chelatable analyte, such as glucose, comprising a quantum dot, a polymer matrix comprising a polymer including moieties that bind the chelatable analyte and a chromophore associated with the polymer matrix that binds to the moieties in the absence of the chelatable analyte. Photons emitted by the quantum dot in an excited state may be absorbed by the chromophore in an unbound state but not by the chromphore in a bound state. The moieties may bind the chelatable analyte and chromophore reversibly and competitively. The moieties may be boronic acids or boronic esters.

One or more components of the sensor, such as the moieties and/or chromophore, may be covalently bound to or associated with the polymer matrix. The sensor particles may further comprise a biocompatible layer.

Also described herein are methods for detecting the presence of a chelatable analyte in a medium using the sensor particles described herein. The chelatable analyte may be glucose and the medium may be selected from water blood, plasma and urine. Also described herein is a method for detecting the presence of a chelatable analyte in an animal. The sensor particle may be implanted in the dermis or epidermis and the chelatable analyte, such as glucose, is monitored.

### BRIEF DESCRIPTION OF THE DRAWINGS

Figure 1. Sensor particle **3** with a. chromophore **2** bound to moiety **1,** wherein the bound chromophore emits photons **4** at one wavelength and b. moiety **1** bound to analyte **5** wherein the unbound chromophore **2** emits photons at a second wavelength **6.**
Figure 2. Sensor particle **3** with a. chromophore **2** bound to moiety **1,** wherein the bound chromophore **2** does not absorb photons **4** emitted by the quantum dot and/or fluorescent dye **7** and b. moiety **1** bound to analyte **5** wherein unbound chromophore **2** absorbs photons **4** emitted by quantum dot and/or fluorescent dye **7.**
Figure 3. Sensor particle **3** with a. chromophore **2** bound to moiety **1,** wherein the bound chromophore absorbs photons **4** emitted by the quantum dot and/or fluorescent dye **7** and b. moiety **1** bound to analyte **5** wherein unbound choromophore **2** absorbs photons **4** emitted by quantum dot and/or fluorescent dye 7.

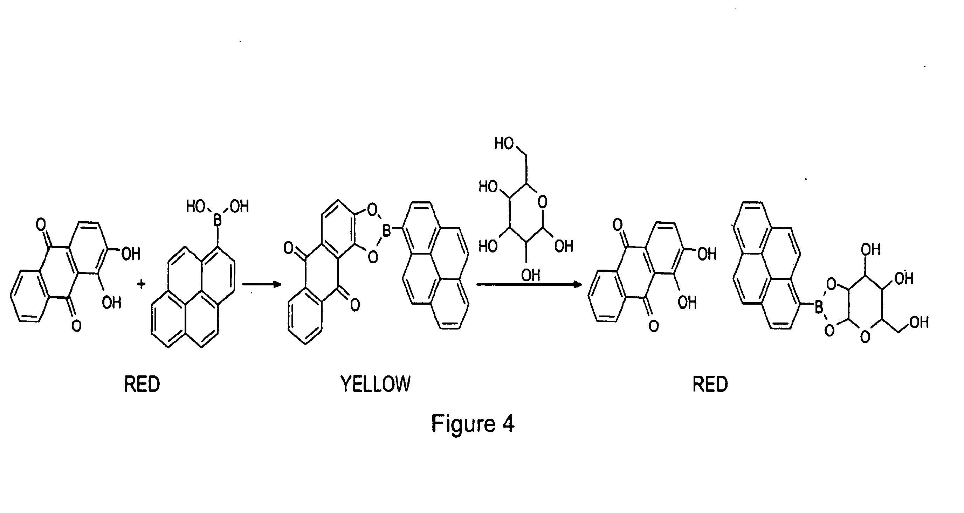

Figure 4. An exemplary embodiment of the competitive interaction of a boronic acid (chelatable moiety) with alizarin (chromophore), or glucose (analyte).

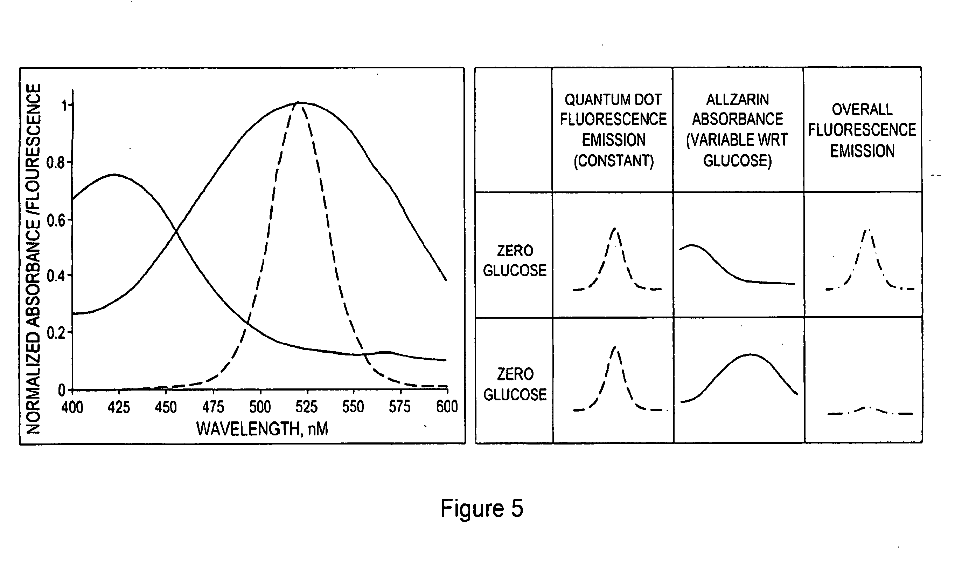

Figure 5. Spectral signature of the components of a GSQD; a. overlap of normalized alizarin absorbance and quantum dot emission, b. individual contribution of the two components of the inner filter effect at high and low glucose concentration and the resulting overall fluorescence signal.
Figure 6. Wide field fluorescence microscopic image of a suspension of sensor particles.
Figure 7. Nanometer-sized sensor particles demonstrating the inner filter effect wherein a. the absorbance changes from purple to yellow depending on the binding state of the chromophore, b. the same samples under UV excitation wherein the sample that was visually purple does not absorb the 525 nm emission of the quantum dots and fluoresces brightly, while the yellow sample absorbs the fluorescence emission of the quantum dot and has minimal emission.
Figure 8. Evaluating response to glucose, the sensor particles containing the essential sensing components, alizarin, pyrene boronic acid and additive, was immobilized to the bottom of a micro-well for calibration. Response to glucose and fructose was measured, the average ± SEM is shown, where n=6 and n=8 for control and monosaccharides, respectively.
Figure 9. Measuring the degree of cytotoxicity of sensor particles by incubating the particles overnight with HEK 293 calls and measuring the degree of cellular injury with an MTT assay. Results of particle sensors are compared to other particles, e.g., gold, latex.

### DETAILED DESCRIPTION

Disclosed are sensor particles for the detection of chelatable analytes, e.g., glucose. The sensor particles comprise a polymer matrix, moieties which bind a chelatable analyte, and a component that emits or absorbs photons of a particular wavelength either in the presence of absence of the chelatable analyte. For exemple, a chromophore absorbs photons of one wavelength when bound to the moieties of the sensor and another wavelength when unbound from the moieties. When the chromophore-bound moieties are exposed to the chelatable analyte, the chromophore is released and the chelatable analyte binds to the moieties. The free chromophore appears as a different color than the bound chromophore, a change which can be monitored visually or with spectrophotometric instrumentation. In another exemple, wherein the inner-filter effect is employed, the sensor particle of the preceding example further comprises a fluorescent dye and/or quantum dot. The fluorescent dye and/or quantum dot absorbs a broad range of wavelengths and emits photons of a narrow range of wavelengths. The fluorescence emitted by the fluorescent component is either absorbed or not absorbed depending on the presence of the chelatable analyte. For example, when the chelatable analyte is bound to the moieties of the sensor, the fluorescence of the quantum dot is absorbed while no absorbance occurs in the absence of the chelatable analyte.

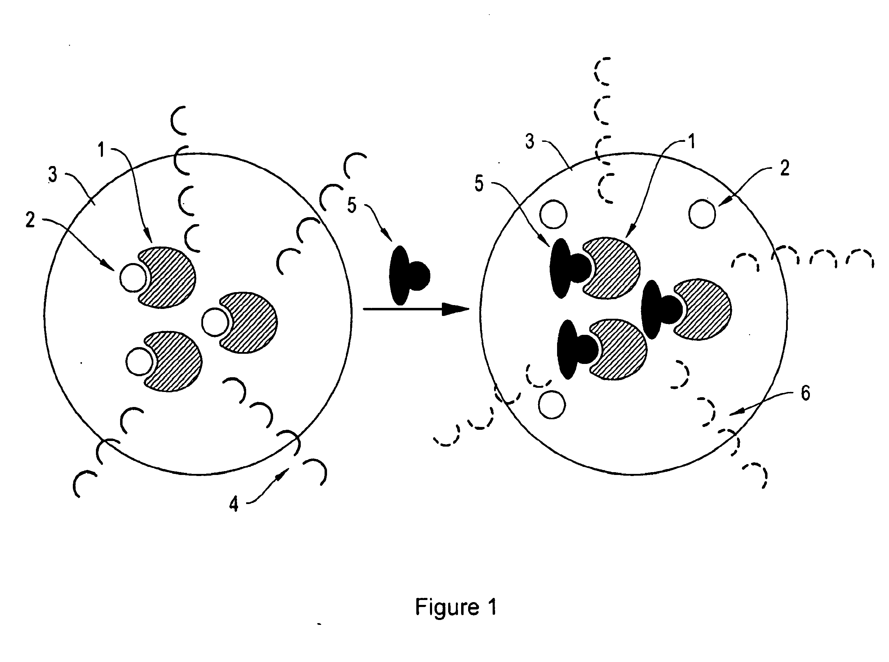

The sensor particle for detecting the presence of chelatable analytes may comprise a polymer matrix comprising a polymer including moieties that bind the chelatable analyte and a chromophore associated with the polymer matrix that binds to the moieties in the absence of the chelatable analyte. The chelatable anaylte may be glucose and the moieties bind glucose and the chromophore reversibly and competitively. For exemple, the sensor particle **3** comprises a polymer matrix with moieties **1** that can bind both a chromophore **2** and glucose **5** (Figure 1). In a first mode, the moieties **1** are bound to a chromophore **2** and the chromophore, in its bound mode, absorbs photons at a first wavelength **4.** In a second mode, when the sensor particle **3** is contacted with glucose **5,** the glucose **5** binds to the moieties **1,** displacing the chromophore **2** which, in its unbound state, absorbs photons at a second wavelength **6**. The sensor **3** may be monitored visually to determine a change in the color of the chromophore **2.** The sensor **3** may be monitored with spectrophotometric instrumentation to determine the emission spectra of the chromophore **2.**

The sensor particle for detecting the presence of a chelatable analyte may comprise a fluorescent component, a polymer matrix comprising a polymer including moieties that bind the chelatable analyte and a chromophore associated with the polymer matrix that binds to the moieties in the absence of the chelatable analyte. The sensor particle may emit photons with an inner filter effect. The inner-filter effect has been documented as a way to increase the signal intensity and concomitant sensitivity of ion-selective optical sensors (optode). In brief, a secondary, inert fluorescent component is added to the polymer matrix of the optode. When the concentration of analyte in the optode changes, the fluorescence intensity of the inert dye itself does not respond, however the absorbance of the sensor does. Because the fluorescence emission has been carefully chosen to overlap with the absorbance spectrum of the sensor, the emission from the inert dye is then absorbed by the sensor. The attenuation of the fluorescence output of the inert dye is therefore directly related to the concentration of the ion of interest in solution.

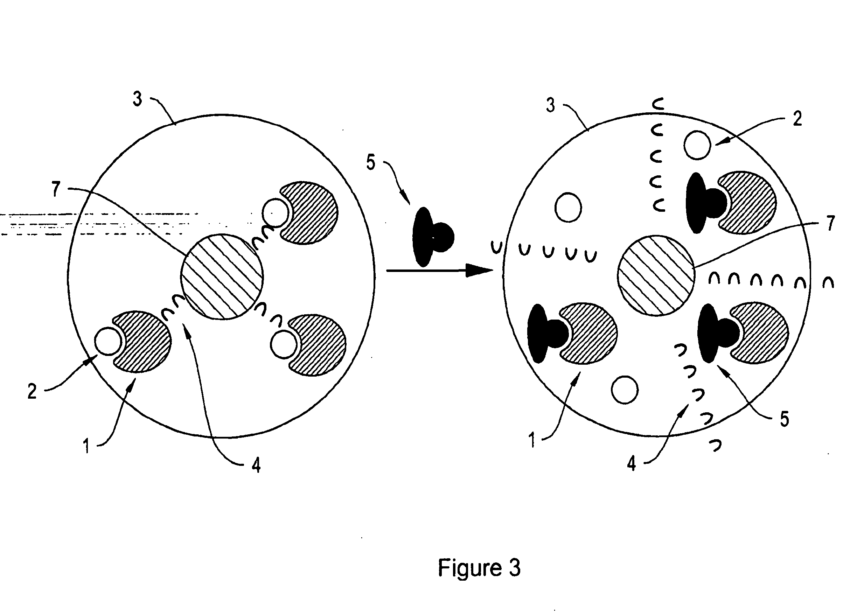

The chelatable analyte may be glucose and the moieties bind glucose and the chromophore reversibly and competitively. The fluorescent component may be selected from one or more quantum dots and/or fluorescent dyes **7**. Here, a sensor particle **3** comprises a fluorescent component 7, and a polymer matrix with moieties **1** that can bind both a chromophore **2** and glucose **5**. The fluorescent component **7** may absorb a broad range of wavelengths of photons but emits a narrow range of wavelengths of photons. The fluorescent component **7** is activated by exciting with a light source, e.g., UV light. The fluorescence emitted from the excited fluorescent component **7** is either absorbed by a component of the sensor, e.g., the chromophore **2** or the glucose-moiety complex, or emitted from the sensor **3** without being attenuated. Photons **4** of the fluorescent component **7** may be absorbed when the chromophore **2** is bound to the moieties **1** (Figure 3, left). Here, the absence of fluorescence emitted from the sensor particle **3** indicates an absence of glucose molecules **5**, i.e. glucose molecules are not bound to the moieties of the sensor. Here, when glucose **5** is introduced, the moieties **1** bind glucose **5,** releasing the chromophore **2.** The photons **4** of the fluorescent component **7** are no longer absorbed by a component of the sensor, Figure 3, right. By detecting the emitted photons, the amount of bound glucose can be calculated relative to a standard.

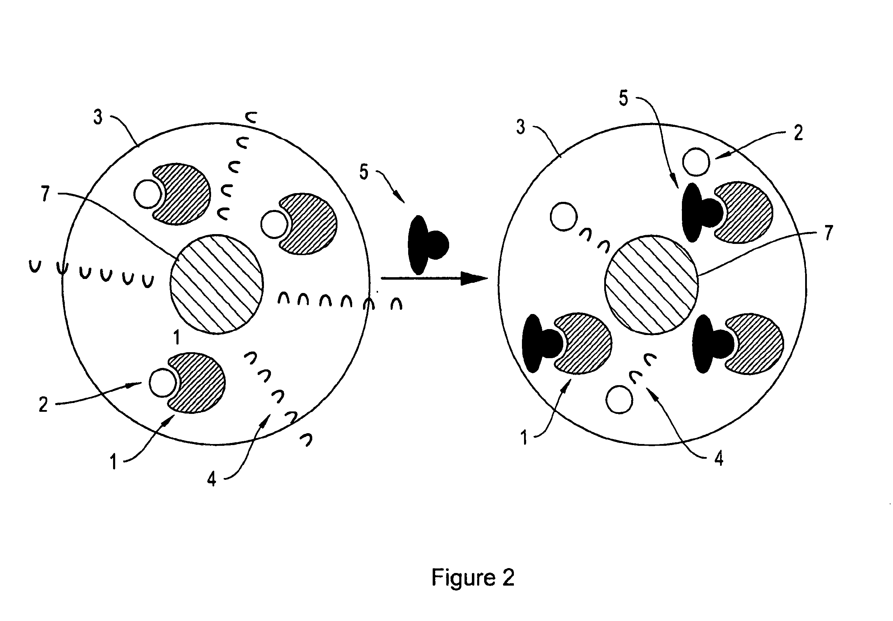

A component of the sensor, e.g., the chromophore **2** or the glucose-moiety complex, may absorb photons **4** of the fluorescent component **7** when unbound from the moieties **2** (Figure 2, right). The detection of photons **4** from the sensor **3** may indicate the absence of glucose **5,** i.e. glucose molecules are not bound to the moieties of the sensor. Here, when the sensor **3** is contacted with glucose **5,** the moieties **1** release the chromophore **2** and bind glucose **5**. Also here, the photons **4** of the fluorescent component **7** may not be absorbed when glucose **5** is bound to the moieties 1 such that the detection of photons 4 emitted from the sensor particle **3** indicates the presence of glucose **5**.

The sensors described herein may be used to detect and measure the presence of a wide variety of chelatable analytes, e.g., sugars and related compounds, in a solution, in vitro or in vivo. The sensor may be located within a cell, i.e., intracellular, or exterior to a cell, i.e., extracellular. The sensor may be in contact with the cell membrane such as within a cell or exterior to a cell. Exemplary chelatable analytes for detection by the sensor described herein include sugars such as glucose, mannose, and other monosaccharides, sialic acid, lactic acids, aminosugars, such as glucosamine, disaccharides, trisaccharides, oligosaccharides, sugar-amino acids, sugar-peptides and glycoproteins. Other exemplary chelatable analytes include, but are not limited to, glycerol, dopamine, catechols, ascorbic acid, polyols, diols such as 1,4-anhydroerythritol and ethylene glycol. The concentration range of chelatable analytes which is typically of interest in biological samples is 0-25 mM, such as from 5-20 mM, such as from 5-10 mM, such as from 0-5 mM.

The moieties that bind the chelatable analytes may comprise a dihydroxide component, e.g., boron and alkali earth dihydroxides. Complexation of sugars, for example, with boron and alkali earth dihydroxides has been reported in, among other sources, [S. A. Barker et al., Carbohydrate Research, 26 (1973) 33-40; N. Roy et al., Carbohydrates Research, 24 (1972) 180-183]. A variety of different boronic acids, having the structure RB(OH)₂ may be used to chelate the analyte. R can be, for example, an aryl or a saturated or unsaturated alkyl moiety, either of which can be substituted or unsubstituted and can contain one or more heteroatoms, e.g., N, S, O, P, B, F, Br. A boronic ester may be used to chelate the analyte. Boronic esters have the molecular formula RB(OR')₂ wherein R' is typically an alkyl group and R can be defined as above. Under aqueous conditions, many boronic esters hydrolyze to form boronic acids. Therefore, OR' groups that hydrolyze to OH are of use in the present disclosure. The two R' groups of the ester may be linked to form a cyclic structure, e.g., -CH₂CH₂-. The moieties may be selected from one ore more aromatic or aliphatic boronic esters. Boronic acids may be appended with substituents that affect the pKa such as electron withdrawing groups or electron donating groups. The pKₐ of the boronic acid may change the dynamic range of the sensor. The dynamic range of the sensor may relate to the affinity for an analyte, such as glucose. The moieties may be selected from one or more aromatic or aliphatic boronic acids. Exemplary boronic acid moieties described herein include phenyl boronic acid, butyl boronic acid, (3,5-dichlomphenyl)boronic acid, [3,5-bis(trifluoromethyl)phenyl]boronic acid, and (4-bromophenyl)boronic acid.

The moieties of the sensor which chelate the analytes may comprise a metal ion. The ability of sugars, for example, and other molecules to form chelate complexes with metal ions in aqueous solution is well known (general review by: Whitfield, D. M. et al., "Metal coordination to carbohydrates. Structure and Function," Coord. Chem. Reviews 122, 171-225 (1993) and Angya, S.J. Complexes of Metal Cations with Carbohydrates in Solution, in "Advances in Carbohydrate Chemistry and Biochemistry," Academic Press, Inc. 1989, pp.1-4). The complexation of Cu(II) with various sugar α-amino acids is described by M. Angeles Diaz-Diez et al., Transition Met. Chem. 20, 402-405, 1995. Sugar-α-amino acid compounds will also form complexes with Co(II), Ni(II), Zn(II) and Cd(II) (M. Angeles Diaz-Diez et al., J. Inorg. Biochem. 56, 243-247, 1994). Additionally, complexes of various sugars with vanadium, molybdenum, tungsten, aluminum, iron, barium, magnesium, and strontium are known (Sreedhara, A. et al., Carbohydrate Res. 264, 227-235, 1994; Caldeira, M. M. et al., Inorg. Chim. Acta. 221, 69-77,1994; Tonkovic, M. and Bilinski, H., Polyhedron 14, 1025-1030, 1995; Nagy, L. et al., Inorg. Chim. Acta. 124,55-59, 1986; Tajmir-Riahi, H. A., Inorg. Chim. Acta. 119,227-232,1986; and Tajmir-Riahi, H. A., J. Inorg. Biochem., 24, 127-136, 1985.

The moieties that bind the chelatable analytes may be covalently, conjugated to the polymer matrix. The moieties may be covalently conjugated to the matrix, for example, through a linker molecule. For exemple, the moieties comprise aryl boronic acids which are covalently conjugated to the polymer matrix through ester linkages originating at an aryl atom or the aryl boronic acid. Other exemplary linkages include amides, ethers, sulfonates, thioethers, thioesters and carbonates. The moieties also may be covalently bound to the polymer matrix through a bond such as a single or double bond. For exemple, the aryl boronic acids are covalently bound to the polymer matrix through a single bond originating from an aryl atom or the aryl boronic acid.

The chromophore of the sensor may be any molecule that binds reversibly to the moieties of the sensor, e.g., the chromophore alizarin binds boronic acids, and absorbs photons of the fluorescent component in a first state and does not absorb photons of the fluorescent component in a second state. The states of the chromophore include bound to the moieties and unbound from the moieties. For example, the chromophore alizarin absorbs at a first wavelength when unbound and a second wavelength when bound to a boronic acid. The chromophore, e.g., alizarin, may be selected from any dye that binds boronic acid moieties, preferably having absorbance/fluorescence properties that differ in the bound vs. the free state. When a suitable chelatable analyte is present, the boronic acid releases the chromophore and binds the analyte. Additional FDA approved dyes and colored drugs are described in the Code of Federal Regulations (CFR) for Food and Drugs (see Title 21 of CFR chapter 1, parts 1-99). A wide variety of chromophores and fluorescence sources may be used, e.g., paired so that the absorbance wavelength of the unbound chromophore substantially matches the wavelength of the fluorescent component's photon emissions, e.g., so as to absorb the emissions in an unbound state. The table below lists a number of suitable chromophores, their Chemical Abstract Service (CAS) Registration Numbers, colors and absorption maxima. The chromophore may be derivatized in such a manner that it can bind with the chelating moiety of the sensor.

| **Chromophore** | **CAS Reg. No.** | **Color** | **Abs. Max.** |
|---|---|---|---|
| Yellow No. 5 | 1934-21-0 | yellow | 428 |
| β-carotene | 7235-40-7 | orange | 466 |
| Rifampin | 3292-46-1 | red | 475 |
| Yellow No. 6 | 2783-94-0 | yellow | 480 |
| Tetracycline | 60-54-8 | yellow | N/A |
| Red No. 40 | 25956-16-6 | red | 502 |
| Red No. 3 | 16423-68-0 | red | 524 |
| Blue No. 2 | 860-22-0 | blue | 610 |
| Evan's blue | 314-13-6 | blue | 610 |
| Green No. 3 | 2353-45-9 | green | 628 |
| Blue No. 1 | 2650-18-2 | blue | 630 |
| Methylene blue | 7220-79-3 | Blue | 668/609 |
| Indocyanine green | 3599-32-4 | Green | 800 (mosdy IR) |

The chromophore may be covalently conjugated to the polymer matrix and comprises a reactive site that binds reversibly with the chelatable analyte selective moieties. For exemple, the chromophore is alizarin, and the alizarin is covalently bound to the polymer matrix through a linker or bond. The linker may be an ester amide, ether, sulfonate, thioether, carbonate or thioester originating from an aromatic carbon of the alizarin. The chromophore may be covalently conjugated through a bond to the polymer matrix. The bond or linkage between the chromophore and the polymer matrix may not interfere with the ability of the chromophore to bind to the chelatable analyte. For example, in the case of alizarin, the linkage or bond to the polymer matrix originates from a ring of the polycyclic ring system that does not bear the hydroxy groups. Here, the hydroxyl groups of the alizarin may be unimpeded from interacting with the chelatable analyte.

The polymer matrix of the sensor may comprise poly(caprolactone) (PCL), ethylene vinyl acetate polymer (EVA), poly(lactic acid) (PLA), poly(L-lactic acid) (PLLA), poly(glycolic acid) (PGA), poly(lactic acid-co-glycolic acid) (PLGA), poly(L-lactic acid-co-glycolic acid) (PLLGA), poly(D,L-lactide) (PDLA), poly(L-lactide) (PLLA), poly(D,L-lactide-co-caprolactone), poly(D,L-lactide-co-caprolactone-co-glycolide), poly(D,L-lactide-co-PEO-co-D,L-lactide), poly(D,L-lactide-co-PPO-co-D,L-lactide), polyalkyl cyanoacralate, polyurethane, poly-L-lysine (PLL), hydroxypropyl methacrylate (HPMA), polyethyleneglycol, poly-L-glutamic acid, poly(hydroxy acids), polyanhydrides, polyorthoesters, poly(ester amides), polyamides, poly(ester ethers), polycarbonates, silicones, polyalkylenes such as polyethylene, polypropylene, and polytetrafluoroethylene, polyalkylene glycols such as poly(ethylene glycol) (PEG), polyalkylene oxides (PEO), polyalkylene terephthalates such as poly(ethylene terephthalate), polyvinyl alcohols (PVA), polyvinyl ethers, polyvinyl esters such as polyvinyl acetate), polyvinyl halides such as poly(vinyl chloride) (PVC), polyvinylpyrrolidone, polysiloxanes, polystyrene (PS), polyurethanes, derivatized celluloses such as alkyl celluloses, hydroxyalkyl celluloses, cellulose ethers, cellulose esters, nitro celluloses, hydroxypropylcellulose, carboxymethylcellulose, polymers of acrylic acids, such as poly(methyl(meth)acrylate) (PMMA), poly(ethyl(meth)acrylate), poly(butyl(meth)acrylate), poly(isobutyl(meth)acrylate), poly(hexyl(meth)acrylate), poly(isodecyl(meth)acrylate), poly(laury](meth)acrylate), poly(phenyl(meth)acrylate), poly(methyl acrylate), poly(isopropyl acrylate), poly(isobutyl acrylate), poly(octadecyl acrylate) (jointly referred to herein as "polyacrylic acids"), and copolymers and mixtures thereof, polydioxanone and its copolymers, polyhydroxyalkanoates, poly(propylene fumarate), polyoxymethylene, poloxamers, poly(ortho)esters, poly(butyric acid), poly(valeric acid), poly(lactide-co-caprolactone), trimethylene carbonate, polyvinylpyrrolidone, and the polymers described in Shieh et al., 1994, J. Biomed. Mater. Res., 28, 1465-1475, and in U.S. Patent No. 4,757,128, Hubbell et al., U.S. Pat. Nos. 5,654,381; 5,627,233; 5,628,863; 5,567,440; and 5,567,435. Other suitable polymers include polyorthoesters (e.g. as disclosed in Heller et al., 2000, Eur. J. Pharm. Biopharm., 50:121-128), polyphosphazenes (e.g. as disclosed in Vandorpe et al., 1997, Biomaterials, 18:1147-1152), and polyphosphoesters (e.g. as disclosed in Encyclopedia of Controlled Drug Delivery, pp. 45-60, Ed. E. Mathiowitz, John Wiley & Sons, Inc. New York, 1999), as well as blends and/or block copolymers of two or more such polymers. The carboxyl termini of lactide- and glycolide-containing polymers may optionally be capped, e.g., by esterification, and the hydroxyl termini may optionally be capped, e.g., by etherification or esterification. In certain embodiments, the polymer comprises or consists essentially of polyvinyl chloride (PVC), polymethyl methacrylate (PMMA) or decyl methacrylate or copolymers or any combination thereof.

The polymer matrix of the sensor may comprise a biocompatible layer, e.g., selected from poly(caprolactone) (PCL), ethylene vinyl acetate polymer (EVA), poly(ethylene glycol) (PEG), poly(vinyl acetate) (PVA), poly(lactic acid) (PLA), poly(glycolic acid) (PGA), poly(lactic-co-glycolic acid) (PLGA), polyalkyl cyanoacrylate, polyethylenimine, dioleyltrimethyammoniumpropane/dioleyl-sn-glycerolphosphoethanolamine, polysebacic anhydrides, polyurethane, nylons, or copolymers thereof. The biocompatible layer may be disposed on the exterior of the sensor such as disposed around the polymer matrix and chromophore and optional component, such as a fluorescent dye and/or quantum dot In polymers including lactic acid monomers, the lactic acid may be D-, L-, or any mixture of D- and L- isomers. The biocompatible layer of the sensor particle may comprise a PEG-lipid. The lipid tail may self-insert into the lipophilic polymer matrix during fabrication, leaving the PEG headgroup on the surface of the sensor, e.g., to provide a hydrophilic, biocompatible coating that can be penetrated by the analyte. Different chemical moieties, such as amines, can be put on the surface or further modified to attach antibodies or other recognition units.

The terms "biocompatible polymer," "biocompatible layer" and "biocompatibility" when used in relation to polymers are art-recognized. For example, biocompatible polymers include polymers that are neither themselves toxic to the host (e.g., a cell or an animal such as a human), nor degrade (if the polymer degrades) at a rate that produces monomeric or oligomeric subunits or other byproducts at toxic concentrations in the host. Consequently, toxicology of a biodegradable polymer intended for intracellular and/or in vivo use, such as implantation or injection into a patient, may be determined after one or more toxicity analyses. It is not necessary that any subject composition have a purity of 100% to be deemed biocompatible. Hence, a subject composition or layer may comprise 99%, 98%, 97%, 96%, 95%, 90% 85%, 80%, 75% or even less of biocompatible polymers, e.g., including polymers and other materials and excipients described herein, and still be biocompatible.

The polymer matrix of the sensor may comprise a plasticizer, such as dioctyl sebacate (DOS), o-nitrophenyl-octylether, dimethyl phthalate, dioctylphenylphosphonate, dibutyl phthalate, hexamethylphosphoramide, dibutyl adipate, dioctyl phthalate, diundecyl phthalate, dioctyl adipate, dioctyl sebacate, or other suitable plasticizers. In certain embodiments, the plasticizer is poly(glycerol sebacate), PGS.

E.g., particularly where the polymer is biocompatible, a biocompatible plasticizer is used. The term "biocompatible plasticizer" is art-recognized, and includes materials which are soluble or dispersible in the relevant polymer, which increase the flexibility of the polymer matrix, and which, in the amounts employed, are biocompatible. Suitable plasticizers are well known in the art and include those disclosed in U.S. Pat. Nos. 2,784,127 and 4,444,933. Specific plasticizers include, by way of example, acetyl tri-n-butyl citrate (c. 20 weight percent or less), acetyltrihexyl citrate (c. 20 weight percent or less), butyl benzyl phthalate, dibutylphthalate, dioctylphthalate, n-butyryl tri-n-hexyl citrate, diethylene glycol dibenzoate (c. 20 weight percent or less) and the like.

The sensor particle for detecting the presence of glucose may comprise: a quantum dot, a polymer matrix comprising a polymer appended with moieties that selectively bind glucose, a chromophore associated with the polymer matrix that binds the moieties in the absence of glucose and a biocompatible layer.

Additives to the polymer matrix may make the extraction of the analyte (e.g., glucose) into the polymeric matrix more efficient. The addition of amine-based additives to the matrix may lower the effective dynamic range of the sensor particles. The addition of amines to the polymer matrix may increase the affinity of the polymer matrix for the analyte, e.g., glucose.

The sensor may comprise one or more quantum dots. Quantum dots are fluorescent semiconductor nanocrystals having a characteristic spectral emission, which is tunable to a desired energy by selection of the particle size, size distribution and composition of the semiconductor nanocrystal. The quantum yield of quantum dots is high, with reports of greater than 90% efficiency in cladded quantum dots, photobleaching is minimal, and a single quantum dot can be continuously tracked for minutes to hours. There is a wide range of colors available, all with the same excitation wavelengths, and very narrow emission bandwidths.

The emission spectra of a population of quantum dots have linewidths as narrow as 25-30 nm, depending on the size distribution heterogeneity of the sample population, and lineshapes that are symmetric, gaussian or nearly gaussian with an absence of a tailing region. Advantageously, the range of excitation wavelengths of the quantum dots is broad. Consequently, this allows the simultaneous excitation of varying populations of quantum dots in a system having distinct emission spectra with a single light source, e.g., in the ultraviolet or blue region of the spectrum.

Quantum dots of the sensor described herein are, for example, inorganic crystallites between 1 nm and about 1000 nm in diameter, preferably between about 2 nm and about 50 nm, more preferably about 5 nm to 20 nm, such as about 6, 7, 8, 9, 10, 11, 12, 13, 14, 15, 16, 17, 18,19, or 20 nm. Such quantum dots include a "core" of one or more first semiconductor materials, and which may be surrounded by a "shell" of a second semiconductor material. A semiconductor nanocrystal core surrounded by a semiconductor shell is referred to as a "core/shell" semiconductor nanocrystal. The surrounded "shell" will most preferably have a bandgap greater than the bandgap of the core material and can be chosen so to have an atomic spacing close to that of the "core" substrate. The core and/or the shell material can be a semiconductor material including, but not limited to, those of the group II-VI (ZnS, ZnSe, ZnTe, CdS, CdSe, CdTe, HgS, HgSe, HgTe, MgTe and the like) and III-V (GaN, GaP, GaAs, GaSb, InN, InP, InAs, InSb, AlAs, AlP, AlSb, AlS, and the like) and IV (Ge, Si, Pb and the like) materials, and an alloy thereof, or a mixture thereof

A sensor may comprise exactly one quantum dot. A sensor may comprise more than one quantum dot, for example, 2, 3, 4, or 5 quantum dots. If the sensor comprises more than one quantum dot, the sensor may comprise two or more types of quantum dots, each type having a distinct emission wavelength, e.g., independently selected from, for example, 490, 520, 545, 560, 580, 620,655 nm. The availability of two distinct wavelength emissions (e.g., one or more quantum dots of wavelength 545 nm and one or more quantum dots with emission wavelength of 655 nm) may allow improvements in recording of changes in analyte concentration by using the ratio of the two distinct signals. Fluctuations in fluorescence that are common to both signals should theoretically cancel in a ratio. The detectable fluorescence emission of the quantum dot particles may fluctuate depending on variables including number of quantum dots, quantum dot location within the cell, photobleaching, and possible changes in excitation light intensity, all effects that can occur slowly and are not related to analyte presence or concentration. Therefore, effects including number of quantum dots, quantum dot location within the cell, photobleaching, and possible changes in excitation light intensity, may be attenuated.

The fluorescence signal of the quantum dot may trigger a detectable event within the cell. For example, fluorescence may in turn excite a secondary dye or quantum dot in the particle that easily generates reactive oxygen species (ROS). The ROS would then attack the cell, effectively stimulating necrosis (cell death), which may then be detected either visually or using markers sensitive to cell death. Alternatively, instead of including a secondary component within the particle, another particle may be added to the cell or cell culture. This additional particle may, for example, comprise a photo-degradable polymer membrane. When the fluorescent component fluoresces, the emitted light will rupture the secondary particle, releasing its contents. The contents may, for example, be a drug that is therapeutic or apoptotic, e.g., triggering another detectable event.

The sensor of the application may be a polymer film. The film may comprise a polymeric matrix comprising a fluorescent component, a chromophore and moieties that chelate analytes. The film may comprise multiple fluorescent components, chromophores and moieties that chelate analytes. The film may be a polymer matrix comprising one of more sensor particles described herein. A sensor film may be deposited on any surface such as plastic, metal, paper or glass. The film may be deposited on an item such as a multi-well plate, a stirring rod, a Petri dish or sample cup. The film can be applied to a surface such as by painting or spraying the surface with the polymer film, or by immersing the surface in a solution or dispersion of the elements of the polymer film. The polymer film may solidifie after the film has been applied to the surface. The polymer used in such films may be any one or more of the polymers described herein or any other suitable polymer. The film may further comprise a biocompatible coating. The fluorescent component of the film may be one or more quantum dots.

The quantum dot of the sensor particle may be modified with a surface modifier, e.g., to alter one or more properties of the sensor particle, such as solubility, biocompatibility, or hydrophilicity/hydrophobicity. The surface modifier may comprise one or more ligands that can bind reversibly with the quantum dot, while also, the surface modification may be essentially irreversible. The surface modifier may improve the lipophilicity of the quantum dot. The ligand may comprise an alkane such as decane-thiol.

Also described herein are methods of preparing particles selective for a chelatable analyte, comprising contacting a quantum dot with a polymeric precursor mixture including moieties that bind the chelatable analyte, and a chromophore. Moieties may be chosen which chelate glucose. The moieties that bind the chelatable analytes may comprise boronic acids and/or boronic esters. The method may further comprise coating the polymer matrix with a biocompatible layer.

Chemical Vapor Deposition (iCVD), a coating technology, may be used to deposit a layer that protects the sensors from the surrounding medium. The solventless nature ofiCVD particle coating may offer an advantage over solution-based methods that rely on drying of a wet polymer solution. The iCVD particle coating may employ a custom-designed rotating bed reactor that has been demonstrated to provide conformal coating of microspheres and nanoparticles without inducing aggregation. The primary monomer for the iCVD coatings of GSQDs may be hydroxyethylmethacrylate (HEMA) monomer.

The iCVD coatings of the nanoparticles may be pure polymer and no residual solvent is present, e.g., that may cause implant rejection, irritation, or other unwanted side effects. The coatings can be applied at room temperature in a single step, taking only a few minutes of total time. The composition can be controlled systematically by changing the gas feed mix and thickness can be controlled by *in situ* monitoring

Also described herein are methods for detecting the presence of a chelatable analyte in a medium, comprising contacting a sensor particle of the invention with a medium, exposing the quantum dot to light energy that causes the quantum dot to emit photons and using a detector to detect the photons and determining the presence or absence of bound chelatable analyte based on the detected photons. The chelatable analyte may be glucose. In certain embodiments, the light energy is selected from ultraviolet, infrared, near infrared or visible radiation. The light energy may be ultraviolet. The medium may comprise water, blood, plasma or urine. The method of detecting glucose with a sensor particle described herein may be performed *in vitro*.

Also described herein is a method for detecting an analyte in an animal using any of the sensor particles described herein. Also described herein is a method for detecting the presence of a chelatable analyte in an animal, comprising the steps of: contacting a sensor particle described herein with an animal cell or tissue, wherein the sensor particle comprises at least one quantum dot and/or fluorescent dye; a polymer matrix comprising a polymer matrix including moieties that bind a chelatable analyte and a chromophore associated with the polymer matrix that binds to the moieties in the absence of the chelatable analyte; exposing the particles to light energy that causes the quantum dot and/or fluorescent dye to emit photons; using a detector to detect the photons; and determining the presence or absence of bound chelatable analyte based on the detected photons. The particle may be implanted within the dermis or epidermis of an animal. The chelatable analyte may be glucose.

The particle may comprise a biocompatible layer. The term "particle" may refer to one or more sensor particle described herein. The particle may comprise many sensor particles. The particle may comprise a fluorescent dye and/or a quantum dot. The particle may comprise at least one quantum dot, a chromophore, and a polymer matrix. The photons emitted by the quantum dot in an excited state may be absorbed by a chromophore in an unbound state but not absorbed by a chromophore in a bound state. The photons emitted by the quantum dot in an excited state may be absorbed by a chromophore in a bound state but not absorbed by a chromophore in an unbound state.

The method for detecting an analyte in an animal may comprise implanting the particle below the surface of the epidermis or dermis of the animal. The particle may be implanted intracellularly, while in other embodiments, the sensors are implanted extracellularly. When implanted in tissues, the composition may be taken into a cell or remain external to a cell. The particle may be implanted between about 0.05 mm and about 4 mm below the surface of the epidermis or dermis of the animal. The particle may be injected or surgically inserted within the dermis or epidermis of an animal. The particle may be injected within the dermis or epidermis of the animal. In certain embodiments, the particle is injected in a solution. A particle solution may comprise multiple particles. The particle solution may comprise particles with an average particle size between 10 nm and 10 microns. The particle solution may comprise particles with an average particle size between 10 microns and 500 microns such as between 50 microns and 200 microns. The amount of signal may decrease over time due to fouling and leaching for the implanted particle sensor is minimal.

The implanted particle may produce an optical change upon contact with a chelatable analyte. The optical change may be the appearance of a color upon chelation of the moieties of the particle with the chelatable analyte. For example, when a colorless particle comes into contact with the chelatable analyte glucose, the chelatable particle turns red. If the particle is implanted in the dermis or epidermis, the color change can be seen from the surface of the skin. Also, the sensor may turn yellow, green, blue, purple or orange.

The particle may emit protons when contacted by a chelatable analyte which can be detected spectrophotometrically. The particle may emit photons immediately upon making contact with the chelatable analyte. The particle may emit photons after a brief time such as 1-5 seconds upon making contact with the chelatable analyte. For exemple, when a particle comprising a quantum dot contacts glucose, the particle emits photons which can be detected with a spectrophotometer. The number of photons detected can be correlated with the amount of chelatable analyte present in a medium, e.g., blood. If the particle is implanted in the dermis or epidermis, the photons can be detected through the skin. The detector may be a hand held unit that can be held near the skin to detect photons emitted from the sensor.

The epidermis may vary in thickness depending upon its location and the animal, but is generally up to about 1 mm thick in a human. When implanted in the epidermis, it is preferred that the particle is placed or implanted of from about 0.05 mm, about 0.06 mm, about 0.07 mm, about 0.08 mm, about 0.09 mm, about 0.10 mm, about 0.12 mm, about 0.14 mm, about 0.16 mm, about 0.18 mm, about 0.2 mm, about 0.22 mm, about 0.24 mm, about 0.26 mm, about 0.28 mm, about 0.30 mm, about 0.32 mm, about 0.34 mm, about 0.36 mm, about 0.38 mm, about 0.40 mm, about 0.42 mm, about 0.44 mm, about 0.46 mm, about 0.48 mm, about 0.50 mm, about 0.52 mm, about 0.54 mm, about 0.56 mm, about 0.58 mm, about 0.60 mm, about 0.62 mm, about 0.64 mm, about 0.66 mm, about 0.68 mm, about 0.70 mm, about 0.72 mm, about 0.74 mm, about 0.76 mm, about 0.78 mm, about 0.80 mm, about 0.82 mm, about 0.84 mm, about 0.86 mm, about 0.88 mm, about 0.90 mm, about 0.92 mm, about 0.94 mm, about 0.96 mm, or about 0.98 mm to about 1 mm below the outer surface of the epidermis of an animal. Also the particle may be implanted between about 0.1 mm and about 0.15 mm below the surface of the epidermis of the animal. Preferred animals include sheep, goats, cats, dogs, birds, cows, horses or pigs. A particularly preferred animal is a human.

When implanted in the epidermis of an animal, the particle may exist only days or weeks before the cells containing or surrounding the particle are shed from the animal. The particle may remain in the position in which it was implanted for 1-4 weeks. The particle may exist up to about 2 weeks before removal through natural replacement of epidermal layers.

The particle is may be implanted in the dermis or dermal layers of an animal. The dermis may very in thickness depending upon its location and the animal, but is generally from about 1 mm to about 4 mm thick in a human. The dermis is located beneath the epidermis, often generally beginning about 1 mm beneath the epidermis, often generally beginning about 1 mm beneath the outer surface of the epidermis. The dermis does not actively shed, so that a particle may exist semi-permanently or permanently in an animal, i.e., remain in the dermis for months or years. Depending on the thickness of the epidermis and dermis, the particle may be implanted or placed in the dermis of from about 1 mm, about 1.1 mm, about 1.2 mm, about 1.3 mm, about 1.4 mm, about 1.5 mm, about 1.6 mm, about 1.7 mm, about 1.8 mm, about 1.9 mm, about 2.0 mm, about 2.1 mm, about 2.2 mm, about 2.3 mm, about 2.4 mm, about 2.5 mm, about 2.6 mm, about 2.7 mm, about 2.8 mm, about 2.9 mm, about 3.0 mm, about 3.1 mm, about 3.2 mm, about 3.3 mm, about 3.4 mm, about 3.5 mm, about 3.6 mm, about 3.7 mm, about 3.8 mm, about 3.9 mm, about 4.0 mm, about 4.1 mm, about 4.2 mm, about 4.3 mm, about 4.4 mm, about 4.5 mm, about 4.6 mm, about 4.7 mm, about 4.8 mm, or about 4.9 mm to about 5.0 mm beneath the outer surface of the epidermis. The particle could be implanted of from about 1 mm to about 5 mm beneath the surface of the epidermis, with about 2 mm to about 3 mm being particularly preferred.

The particle sensor may be coupled with an optical readout (e.g., placed over the implantation site). A small insulin pump may be coupled to the optical readout device. The insulin pump may be configured such that the insulin pump is activated to deliver insulin if the optical readout detects a level of glucose above a predetermined value.

### EXAMPLES

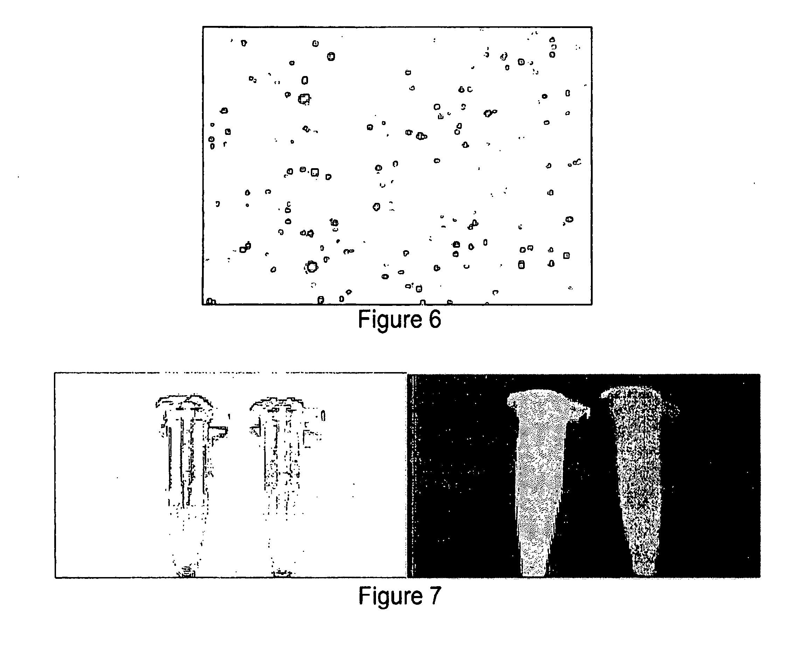

***Nano-scale polymer-coated** quantum dots:* Commercially available quantum dots (Evident Technologies, Troy, NY) were dispersed in a polymeric matrix. In order to make the dispersion homogeneous, a ligand exchange was performed to add a decane-thiol to the surface of the quantum dot. The alkylated surface proved more miscible with the lipophilic polymer matrix. After a homogeneous distribution was obtained, nanoscale sensors were produced by sonicating the polymeric matrix dissolved in THF, containing all of the sensing elements including quantum dots, in an aqueous solution of PEG-lipid surface modifier. The resulting nanosensor solution was filtered to remove larger pieces of polymer. The resulting sensor suspension fluoresced brightly when viewed in a wide-field fluorescence microscope (Figure 6).

***Inner-filter effect:*** Nanometer-sized glucose-sensitive quantum dots (GSQDs) in solution are shown in Figure 7. The absorbance changes from purple to yellow are easily seen by eye in Figure 7 (left). The same samples of nanosensors under UV excitation are shown in Figure 7 (right). The sample that was visually purple does not absorb the 525 nm emission of the quantum dots and fluoresces brightly. The yellow GSQD absorbs the fluorescence emission of the quantum dot and has minimal emission.

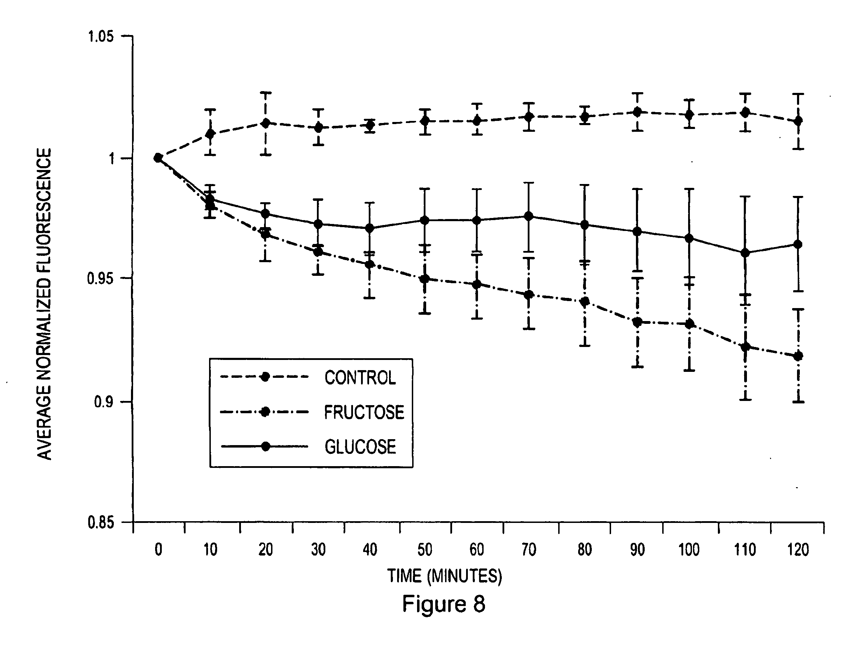

***Response to glucose:*** A polymer matrix containing the sensing components alizarin, pyrene boronic acid and additive, was immobilized to the bottom of a micro-well for calibration. Response to glucose and fructose was measured, the average ± SEM is shown in Figure 8, n = 6 and 8 for control and monosaccharides, respectively.

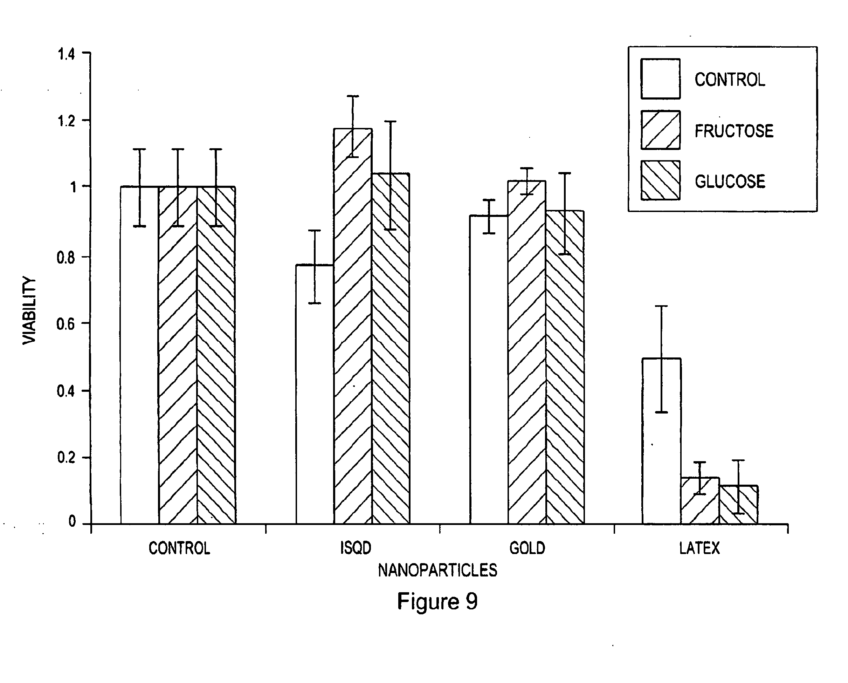

***Biocompatibility:** In vitro* biocompatibility studies produced no indications of cellular injury thus far. For instance, LIVE-DEAD assays showed no differences from controls in the amount of cell death. In addition, the degree of cytotoxicity was determined by incubating the nanosensors overnight with HEK 293 cells and measuring the degree of cellular injury with an MTT assay. These results were compared to other nanoparticles and are shown in Figure 9. The ion-sensitive quantum dot (ISQD) nanosensors show no cellular toxicity compared to controls over the course of 72 hours after incubation. This result is also seen for 100 nm diameter gold nanoparticles,

### EQUIVALENTS

The present invention provides among other things sensor particles for detecting chelatable analytes and methods of use thereof. While specific embodiments of the subject invention have been discussed, the above specification is illustrative and not restrictive. Many variations of the invention will become apparent to those skilled in the art upon review of this specification. The full scope of the invention should be determined by reference to the claims, along with their full scope of equivalents, and the specification, along with such variations.

## Claims

1. A sensor particle for detecting the presence of a chelatable analyte, comprising:
a quantum dot or fluorescent dye;
a polymer matrix comprising a polymer including moieties that bind the chelatable analyte, wherein the chelatable analyte is glucose or fructose; and
a chromophore associated with the polymer matrix that binds to the moieties in the absence of the chelatable analyte, wherein the moieties that bind glucose or fructose comprise one of boronic acid and boronic ester; and the sensor particle emits photons with an inner filter effect, wherein: photons emitted by the quantum dot or fluorescent dye in an excited state are absorbed by the chromophore in an unbound state but not by the chromophore in a bound state; or
photons emitted by the quantum dot or fluorescent dye in an excited state are absorbed by the chromophore in a bound state but not by the chromophore in an unbound state.

2. The particle of claim 1, further **characterized by** one or more of:
a) the chromophore absorbs photons of a first wavelength when bound to the moieties, and absorbs photons of a second wavelength when released from the moieties;
b) the boronic acid or boronic ester moieties are covalently conjugated through linkers to the polymer matrix;
c) the moieties bind the chelatable analyte and the chromophore reversibly and competitively;
d) the chromophore is covalently conjugated to the polymer matrix; and
e) the particle comprises a biocompatible coating disposed on at least a portion of the polymer matrix.

3. The particle of claim 1 or 2, comprising a fluorescent dye.

4. The particle of claim 1 or 2, comprising a quantum dot.

5. A method of preparing sensor particles selective for a chelatable analyte wherein the chelatable analyte is glucose or fructose, comprising contacting a quantum dot or fluorescent dye with a polymeric precursor mixture including moieties, wherein the moieties that bind the chelatable analyte comprise boronic acids and/or boronic esters, that bind the chelatable analyte, and a chromophore selected such that in the sensor, photons emitted with an inner filter effect by the quantum dot or fluorescent dye in an excited state are absorbed by the chromophore in an unbound state but not by the chromophore in a bound state, or photons emitted with an inner filter effect by the quantum dot or fluorescent dye in an excited state are absorbed by the chromophore in a bound state but not by the chromophore in an unbound state.

6. The method of claim 5, further **characterized by** further comprising coating at least a portion of the polymer matrix with a biocompatible layer.

7. The method of claim 5 or 6, comprising a fluorescent dye.

8. The method of claim 5 or 6, comprising a quantum dot.

9. An in vitro method for detecting the presence of a chelatable analyte in a medium, comprising:
contacting a particle of any of claims 1-4 with the medium;
exposing the quantum dot to light energy that causes the quantum dot to emit photons;
using a detector to detect the photons; and
determining the presence or absence of bound chelatable analyte, wherein the chelatable analyte is glucose or fructose, based on the detected photons.

10. The in vitro method of claim 9, further **characterized by** one or more of:
the chelatable analyte is glucose;
the light energy is UV radiation;
the medium comprises water, blood, plasma or urine;
the particle is located inside a cell; and
the particle is located outside a cell.

## Patentansprüche

1. Sensorpartikel zum Nachweis der Anwesenheit eines chelatbildenden Analyten, umfassend:
ein Quantumdot oder einen Fluoreszenzfarbstoff;
eine Polymermatrix, umfassend ein Polymer, das Einheiten beinhaltet, die den chelatbildenden Analyten binden, wobei der chelatbildende Analyt Glukose oder Fruktose ist; und
ein Chromophor, das mit der Polymermatrix assoziiert ist und das an die Einheiten in Abwesenheit des chelatbildenden Analyten bindet, wobei die Einheiten, die Glukose oder Fruktose binden, Borsäure oder Borsäureester umfassen; und das Sensorpartikel Photonen mit einem inneren Filtereffekt emittiert, wobei Photonen, die von dem Quantumdot oder dem Fluoreszenzfarbstoff in einem angeregten Zustand emittiert werden, durch das Chromophor in einem ungebundenen Zustand absorbiert werden, jedoch nicht von dem Chromophor in einem gebundenen Zustand; oder
Photonen, die von dem Quantumdot oder dem Fluoreszenzfarbstoff in einem angeregten Zustand emittiert werden, durch das Chromophor in einem gebundenen Zustand absorbiert werden, jedoch nicht von dem Chromophor in einem ungebundenen Zustand.

2. Partikel nach Anspruch 1, das weiterhin **gekennzeichnet ist durch** eines oder mehrere von:
(a) das Chromophor absorbiert Photonen einer ersten Wellenlänge, wenn es an die Einheiten gebunden ist und Photonen einer zweiten Wellenlänge, wenn es von den Einheiten gelöst ist;
(b) die Borsäure- oder die Borsäureestereinheiten sind kovalent über Linker mit der Polymermatrix verknüpft;
(c) die Einheiten binden den chelatbildenden Analyten und das Chromophor reversibel und kompetitiv;
(d) das Chromophor ist kovalent mit der Polymermatrix verknüpft; und
(e) die Partikel umfassen eine biologisch verträgliche Beschichtung, die zumindest auf einem Teil der Polymermatrix aufgebracht ist.

3. Partikel nach Anspruch 1 oder 2, umfassend einen Fluoreszenzfarbstoff.

4. Partikel nach Anspruch 1 oder 2, umfassend ein Quantumdot.

5. Verfahren zur Herstellung von Sensorpartikeln, die spezifisch für einen chelatbildenden Analyten sind, wobei der chelatbildende Analyt Glukose oder Fruktose ist, umfassend das Inkontaktbringen eines Quantumdots oder eines Fluoreszenzfarbstoffs mit einem polymerischen Vorläufergemisch, das Einheiten beinhaltet, wobei die Einheiten, welche den chelatbildenden Analyten binden, Borsäure und/oder Borsäureester umfassen, die den chelatbildenden Analyten binden, und ein Chromophor, das so ausgewählt ist, dass in dem Sensor Photonen, die mit einem inneren Filtereffekt durch das Quantumdot oder den Fluoreszenzfarbstoff in einem angeregten Zustand emittiert werden, durch das Chromophor in einem ungebundenen Zustand absorbiert werden, jedoch nicht durch das Chromophor in einem gebundenen Zustand, oder Photonen, die mit einem inneren Filtereffekt durch das Quantumdot oder den Fluoreszenzfarbstoff in einem angeregten Zustand emittiert werden, durch das Chromophor in einem gebundenen Zustand absorbiert werden, jedoch nicht durch das Chromophor in einem ungebundenen Zustand.

6. Verfahren nach Anspruch 5, weiterhin **dadurch gekennzeichnet, dass** es ferner das Beschichten mindestens eines Anteils der Polymermatrix mit einer biologisch verträglichen Schicht umfasst.

7. Verfahren nach Anspruch 5 oder 6, umfassend einen Fluoreszenzfarbstoff.

8. Verfahren nach Anspruch 5 oder 6, umfassend ein Quantumdot.

9. In vitro-Verfahren zum Nachweis der Anwesenheit eines chelatbildenden Analyten in einem Medium, umfassend:
Inkontaktbringen eines Partikels nach einem der Ansprüche 1 bis 4 mit dem Medium;
Aussetzen des Quantumdots gegenüber Lichtenergie, die bewirkt, dass das Quantumdot Photonen emittiert;
Verwendung eines Detektors zum Nachweis der Photonen; und
Bestimmung der Anwesenheit oder Abwesenheit von gebundenem chelatbildendem Analyten, wobei der chelatbildende Analyt Glukose oder Fruktose ist, basierend auf den nachgewiesenen Photonen.

10. In vitro-Verfahren nach Anspruch 9, das weiterhin **gekennzeichnet ist durch** eines oder mehrere von:
der chelatbildende Analyt ist Glukose;
die Lichtenergie ist UV-Strahlung;
das Medium umfasst Wasser, Blut, Plasma oder Urin;
das Partikel befindet sich innerhalb einer Zelle; und
das Partikel befindet sich außerhalb einer Zelle.

## Revendications

1. Particule de détecteur pour détecter la présence d'un analyte susceptible de chélation, comprenant :
un point quantique ou un colorant fluorescent ;
une matrice polymère comprenant un polymère contenant des fragments qui se lient à l'analyte susceptible de chélation, dans laquelle l'analyte susceptible de chélation est le glucose ou le fructose ; et
un chromophore associé à la matrice polymère qui se lie aux fragments en l'absence de l'analyte susceptible de chélation, les fragments qui se lient au glucose ou au fructose comprenant un parmi l'acide boronique et un ester boronique ; et la particule de détecteur émettant des photons à effet de filtre interne, dans laquelle :
les photons émis par le point quantique ou le colorant fluorescent à l'état excité sont absorbés par le chromophore à l'état non lié mais pas par le chromophore à l'état lié ; ou
les photons émis par le point quantique ou le colorant fluorescent à l'état excité sont absorbés par le chromophore à l'état lié mais pas par le chromophore à l'état non lié.

2. Particule selon la revendication 1, **caractérisée en outre en ce que** :
a) le chromophore absorbe les photons ayant une première longueur d'onde quand il est lié aux fragments, et absorbe les photons ayant une seconde longueur d'onde quand il est séparé des fragments ;
b) les fragments acide boronique ou ester boronique sont liés par covalence par l'intermédiaire de lieurs à la matrice polymère ;
c) les fragments se lient à l'analyte susceptible de chélation et au chromophore de manière réversible et en mode compétition ;
d) le chromophore est conjugué par covalence à la matrice polymère ; et
e) la particule comprend un revêtement biocompatible agencé sur au moins une partie de la matrice polymère.

3. Particule selon la revendication 1 ou 2, comprenant un colorant fluorescent.

4. Particule selon la revendication 1 ou 2, comprenant un point quantique.

5. Méthode de préparation de particules de détecteur sélectives envers un analyte susceptible de chélation, dans laquelle l'analyte susceptible de chélation est le glucose ou le fructose, comprenant la mise en contact d'un point quantique ou d'un colorant fluorescent avec un mélange polymère précurseur contenant des fragments, les fragments se liant à l'analyte susceptible de chélation comprenant des acides boroniques et/ou des esters boroniques, qui se lient à l'analyte susceptible de chélation, et un chromophore choisi de façon que dans le détecteur, les photons à effet de filtre interne émis par le point quantique ou le colorant fluorescent à l'état excité soient absorbés par le chromophore à l'état non lié mais pas par le chromophore à l'état lié, ou de façon que les photons à effet de filtre interne émis par le point quantique ou le colorant fluorescent à l'état excité soient absorbés par le chromophore à l'état lié mais pas par le chromophore à l'état non lié.

6. Méthode selon la revendication 5, **caractérisée en outre en ce qu'**elle comprend en outre le revêtement au moins partiel de la matrice polymère avec une couche biocompatible.

7. Méthode selon la revendication 5 ou 6, comprenant un colorant fluorescent.

8. Méthode selon la revendication 5 ou 6, comprenant un point quantique.

9. Méthode in vitro pour détecter la présence d'un analyte susceptible de chélation dans un milieu, comprenant :
la mise en contact d'une particule selon l'une quelconque des revendications 1 à 4 avec le milieu ;
l'exposition du point quantique à une énergie lumineuse de façon que le point quantique émette des photons ;
l'utilisation d'un détecteur pour détecter les photons ; et
la détermination de la présence ou de l'absence d'analyte susceptible de chélation lié,
l'analyte susceptible de chélation étant le glucose ou le fructose, sur la base des photons détectés.

10. Méthode in vitro selon la revendication 9, **caractérisée en outre en ce que** :
l'analyte susceptible de chélation est le glucose ;
l'énergie lumineuse est un rayonnement UV ;
le milieu comprend l'eau, le sang, le plasma ou l'urine ;
la particule se trouve à l'intérieur d'une cellule ; et
la particule se trouve à l'extérieur d'une cellule.
